# EUROPÄISCHE PATENTSCHRIFT

(11) **EP 2 078 497 B1**
(45) Veröffentlichungstag und Bekanntmachung des Hinweises auf die Patenterteilung: **01.02.2012**
(21) Anmeldenummer: 08021892.8
(22) Anmeldetag: 17.12.2008
(51) Int. Cl.: A61B 17/00, A61B 17/22

(54) **Medizinisches Instrument**
Medical instrument
Instrument médical

(30) Priorität: 10.01.2008 AT 342008
(43) Veröffentlichungstag der Anmeldung: 15.07.2009
(73) Patentinhaber: AMI Agency for Medical Innovations GmbH, 6800 Feldkirch (AT)
(72) Erfinder: Egle, Walter, 6842 Koblach (AT); Wirbel, Mathias, 6751 Braz (AT)
(74) Vertreter: Hofmann, Ralf U.

(56) Entgegenhaltungen:
- EP-A- 1 864 618
- US-A- 5 330 483
- US-A- 5 397 320
- US-A1- 2002 123 761

## Beschreibung

Die Erfindung bezieht sich auf ein medizinisches Instrument, welches einen Bergebeutel zum Bergen von bei einer Operation, insbesondere endoskopischen Operation, aus einem zu operierenden Körper zu entfernenden Material und ein in einer Anfangsstellung innerhalb des Bergebeutels angeordnetes Netz aufweist.

Medizinische Instrumente zum Bergen von bei einer endoskopischen Operation zu entfernendem Material, beispielsweise der Gallenblase, sind in unterschiedlichen Ausführungsformen bekannt. In einer bekannten Ausführungsform ist ein Beutel (="Bergebeutel") mittels eines Bügels aus einem federelastischen Material am distalen Ende eines Schafts des Instruments gehalten. Im Auslieferungszustand des Instruments ist der Beutel aufgerollt und befindet sich mit dem zusammengedrückten Bügel in einem den Schaft umgebenden Hüllrohr. In diesem Zustand wird der Schaft durch den Trokar eingeführt. Wird das Hüllrohr auf dem Schaft in Richtung zum proximalen Ende des Schafts zurückgezogen, so gelangen der Bügel und der an ihm angehängte Beutel aus dem distalen Ende des Hüllrohrs, sodass sich der Beutel entrollen kann und sich der Bügel unter Ausbildung einer Einfüllöffnung des Beutels auseinanderfaltet. Nach dem Einfüllen des zu bergenden Materials in den Beutel kann dieser geschlossen werden, beispielsweise indem das Hüllrohr wiederum nach distal auf dem Schaft verschoben wird und das distale Ende des Hüllrohrs den Beutel auf dem Bügel zusammenschiebt, wobei der Bügel nach und nach wieder zusammengedrückt wird. Ein weiterer möglicher Verschluss des Beutels kann mittels einer sogenannten "Tabaksbeutelnaht" erreicht werden. Hierbei wird eine in einem Kanal des Beutels, der diesen im Bereich seiner Einfüllöffnung umgibt, geführte Schlaufe zusammengezogen, wobei der Beutel auch vom federelastischen Bügel abgerissen wird.

Ein Nachteil dieser vorbekannten Bergeinstrumente besteht darin, dass im Allgemeinen das zu bergende Material zu große Abmessungen besitzt, um durch den Trokar, durch den das Instrument eingeführt ist, herausgezogen zu werden. Auch ein Herausziehen des Instruments zusammen mit dem Trokar durch die Öffnung in der Körperwand, durch welche der Trokar verläuft, ist häufig nur unter Vergrößerung der Öffnung in der Körperwand möglich. Eine andere Möglichkeit besteht darin, den Beutel wieder teilweise zu öffnen und einen Morzellator (=Zerkleinerer) zur Zerkleinerung des im Beutel sich befindenden Materials einzuführen und das zerkleinerte Material in der Folge mittels eines Saugers abzusaugen. Diese Vorgehensweise ist sehr aufwendig.

Ein chirurgisches Bergeinstrument mit einem mittels eines Federbügels an einem Schaft angehängten Bergebeutel ist beispielsweise aus der US 6 409 733 B1 bekannt. Weiters zeigt die US 5 769 794 A einen separaten Bergebeutel, der nicht an einem Schaft festgelegt ist. Ein weiterer an einem Schaft gehaltener Bergebeutel geht aus der US 5 853 374 A hervor, wobei dieser Bergebeutel eine zum distalen Ende des Instruments hin gerichtete Öffnung aufweist, welche verschließbar ist.

Ein medizinisches Instrument der eingangs genannten Art geht aus der US 5,330,483 hervor. In einem Bergebeutel ist ein schlauchförmiges Netz angeordnet, welches aus einem Material gefertigt ist, das sich unter Einwirkung von Hitze zusammenzieht. Durch thermische Aktivierung des schlauchförmigen Netzes wird im Netz angeordnetes Material, welches aus dem zu operierenden Körper zu entfernen ist, komprimiert oder auch zerdrückt.

EP 1 864 618 A1 offenbart ein medizinisches Instrument gemäß dem Oberbegriff des Anspruchs 1.

Aufgabe der Erfindung ist es ein medizinisches Instrument bereit zu stellen, durch welches das Bergen von bei einer Operation, insbesondere endoskopischen Operation, aus einem zu operierenden menschlichen oder tierischen Körper zu entfernendem Material, insbesondere organischem Material, erleichtert wird. Erfindungsgemäß gelingt dies durch ein medizinisches Instrument mit den Merkmalen des Anspruchs 1.

Beim medizinischen Instrument der Erfindung befindet sich im Bergebeutel ein Netz, welches zum Zerkleinern von zu bergendem Material dient. Zu diesem Zweck wird das Netz durch das Material durchgezogen, wobei die Filamente des Netzes durch das Material durchschneiden und das Material hierbei entsprechend der Maschengröße des Netzes zerschneiden. Das Netz kann somit auch als Schneidnetz bezeichnet werden. Das so zerkleinerte Material kann in der Folge aus dem Körper entfernt werden, beispielsweise mittels einer in das medizinische Instrument integrierten Saugleitung.

Das Netz ist beutelförmig ausgebildet oder es bildet einen Teil eines Beutels, d.h. zumindest ein Abschnitt der Wand des Beutels besteht aus einem Netz. Der vom Netz gebildete oder das Netz aufweisende Beutel ist zunächst im Bergebeutel angeordnet, wenn das zu bergende Material in den Bergebeutel eingefüllt wird. Das zu bergende Material gelangt somit in den vom Netz gebildeten bzw. das Netz aufweisenden Beutel. Das zu bergende Material wird in diesem vom Netz gebildeten oder dieses aufweisenden Beutel gehalten, bis das Netz durch das Material hindurch gezogen worden ist, wodurch das Material zerkleinert wird.

In einer vorteilhaften Ausführungsform der Erfindung ist der Bergebeutel von einem Schaft des medizinischen Instruments gehalten, vorzugsweise mittels eines Bügels aus einem federelastischen Material, welcher ohne Einwirkung einer äußeren Kraft sich derart ringförmig (z.B. annähernd kreisringförmig oder oval) auseinanderfaltet, dass eine Einfüllöffnung des Bergebeutels geöffnet ist. Der Bergebeutel ist hierbei im Bereich seiner Einfüllöffnung über den Großteil seines Umfangs am Bügel gehalten. Der vom Netz gebildete oder das Netz aufweisende Beutel ist innerhalb des Bergebeutels angeordnet und besitzt eine Öffnung, die in die gleiche Richtung wie die Einfüllöffnung des Bergebeutels weist. Vorzugsweise liegt der vom Netz gebildete oder das Netz aufweisende Beutel im Wesentlichen an der Wand des Bergebeutels an, zumindest im Bereich der Einfüllöffnung, um eine möglichst große insgesamte Einfüllöffnung auszubilden.

Zum Schließen des Bergebeutels greift vorzugsweise mindestens ein Zugelement am Bergebeutel an, welches mittels eines Betätigungsteils in Richtung zum proximalen Ende des medizinischen Instruments gezogen werden kann. Das Zugelement kann hierbei durch einen Kanal des Bergebeutels in der Nähe seiner Einfüllöffnung verlaufen. Wenn das Zugelement in Richtung zum proximalen Ende des medizinischen Instruments gezogen wird, so wird der Bergebeutel nach und nach vom Bügel abgerissen und zunehmend geschlossen. Vorteilhafterweise kann er hierbei über einen distalen Endabschnitt des Schaftes gezogen werden, wobei an diesem distalen Endabschnitt ein Kanal mündet, der einen inneren Hohlraum des Schaftes bildet. Im geschlossenen Zustand des Beutels ragt somit der distale Endabschnitt des Schaftes durch eine Restöffnung der zusammengezogenen Einfüllöffnung des Bergebeutels in den Bergebeutel hinein. Mittels eines weiteren Zugelements kann der vom Netz gebildete oder das Netz aufweisende Beutel in den, vorzugsweise am distalen Endabschnitt des Schaftes stirnseitig mündenden, Hohlraum hineingezogen werden, wobei das Netz durch zunächst im Netz angeordnetes Material unter Zerschneidung desselben gezogen wird.

In der vorliegenden Schrift beziehen sich die Angaben "proximal" und "distal" auf die Lage zum Benutzer (=Operateur). Ein distales Ende ist somit vom Benutzer entfernt und abgewandt und ein proximales Ende diesem zugewandt.

Weitere Vorteile und Einzelheiten der Erfindung werden im Folgenden anhand der beiliegenden Zeichnung erläutert. In dieser zeigen:
Fig. 1 eine Schrägsicht eines Ausführungsbeispiels eines medizinischen Instruments gemäß der Erfindung, in einem Auslieferungszustand;
Fig. 2 eine Schrägsicht des Instruments von Fig. 1 nach dem Zurückziehen des äußeren Hüllrohrs (=Befüllzustand des medizinischen Instruments);
Fig. 3 eine vergrößerte Schrägsicht eines Teils des Instruments im Zustand von Fig. 2, der Bergebeutel und das Netz mittig aufgeschnitten, ein zu bergendes Material schematisch eingezeichnet;
Fig. 4 einen distalen Abschnitt des Instruments aus einem gegenüber Fig. 3 geänderten Blickwinkel, der Bergebeutel und das Netz über einen Winkelbereich von 90° aufgeschnitten;
Fig. 5 eine Schrägsicht entsprechend Fig. 4, aber ohne den Bergebeutel und das Netz;
Fig. 6 und 7 Darstellungen entsprechend den Fig. 2 und 3, kurz nach Beginn des Schließens des Bergebeutels;
Fig. 8 und 9 Darstellungen entsprechend Fig. 6 und 7, aber der Bergebeutel vollständig geschlossen;
Fig. 10 und 11 Darstellungen entsprechend Fig. 8 und 9, aber das Netz teilweise in den Kanal des Schaftes zurückgezogen;
Fig. 12 und 13 Darstellungen entsprechend Fig. 10 und 11, aber das Netz vollständig in den Kanal des Schaftes zurückgezogen;
Fig. 14 und 15 Darstellungen entsprechend Fig. 12 und 13, aber eine Saugleitung in den Bergebeutel eingefahren.

Die Fig. weisen unterschiedliche Maßstäbe auf.

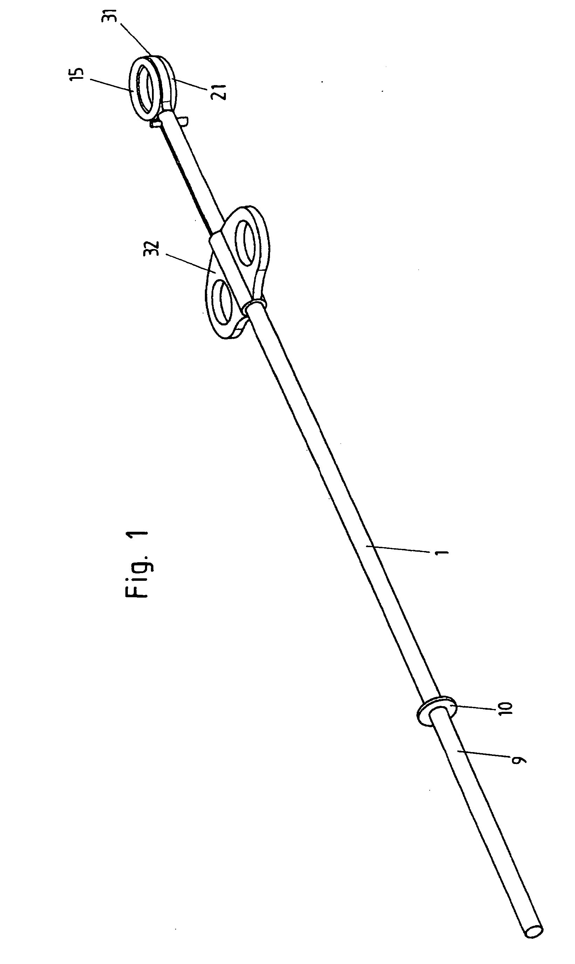

Ein Ausführungsbeispiel eines medizinischen Instruments gemäß der Erfindung ist in den Figuren 1 bis 15 dargestellt. Das Instrument weist einen Schaft 1 auf, an dem im Bereich seines distalen Endes ein Bergebeutel 2 gehalten ist. Der Bergebeutel 2 besitzt ein geschlossenes Ende 3 und eine Einfüllöffnung 4. Der Bergebeutel 2 ist mittels eines Bügels 5 aus einem federelastischen Material am Schaft 1 befestigt. Hierzu sind die beiden Enden des Bügels 5 am Schaft befestigt und der Bügel 5 verläuft durch einen Kanal 6 im Bergebeutel 2, der im Bereich der Einfüllöffnung 4 diese umgebend angeordnet ist. Ohne einwirkende äußere Kraft verläuft der Bügel 5 ringförmig, wobei er die Einfüllöffnung 4 des Bergebeutels 2 öffnet.

Am Schaft 1 kann proximal ein Griff 32 angebracht sein.

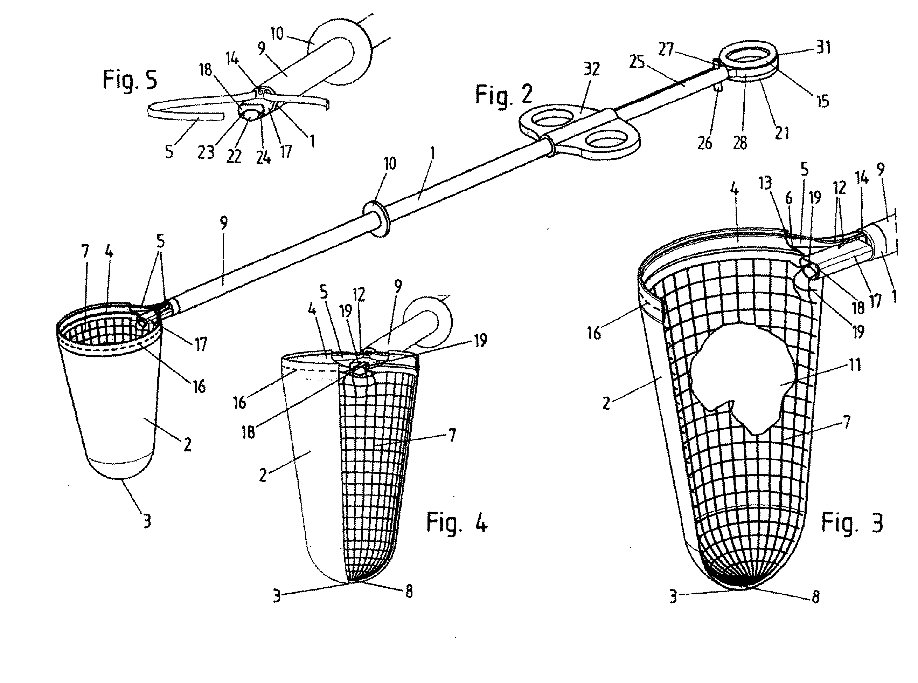

Im Bergebeutel 2 befindet sich ein Netz 7, welches die Form eines Beutels aufweist (=einen Beutel ausbildet). Es befindet sich somit der vom Netz 7 ausgebildete Beutel innerhalb des Bergebeutels 2, wobei der vom Netz 7 gebildete Beutel ein geschlossenes Ende 8 aufweist, das auf der gleichen Seite wie das geschlossene Ende 3 des Bergebeutels 2 liegt, und ein offenes Ende aufweist, welches auf der gleichen Seite wie die Einfüllöffnung 4 des Bergebeutels 2 liegt. Der vom Netz 7 gebildete Beutel liegt in einem Befüllzustand des medizinischen Instruments (vgl. Fig. 2 bis 4) zumindest im Bereich der Einfüllöffnung des Bergebeutels 2 an der inneren Wandung des Bergebeutels 2 an. Dadurch wird eine möglichst große insgesamte Einfüllöffnung ausgebildet und es wird verhindert, dass Material in den Bereich zwischen das Netz 7 und den Bergebeutel 2 gelangen kann. Vorzugsweise liegt der vom Netz 7 gebildete Beutel im Wesentlichen vollständig oder zumindest großteils an der inneren Wandung des Bergebeutels 2 an, um ein möglichst großes insgesamtes Füllvolumen auszubilden.

In einem Auslieferungszustand des medizinischen Instruments (vgl. Fig. 1) befindet sich der Bergebeutel 2 und somit auch das innerhalb des Bergebeutels 2 angeordnete Netz im Inneren eines Hüllrohres 9. Das Hüllrohr 9 ist verschiebbar auf dem Schaft 1 angeordnet. Der Bügel 5 ist zusammengedrückt und der Bergebeutel 2 mit dem sich in ihm befindenden Netz 7 ist zusammengerollt.

Wird das Hüllrohr 9 in Richtung zum proximalen Ende 31 des Instruments auf dem Schaft 1 verschoben, so gibt es nach und nach den Bügel 5 mit dem von ihm gehaltenen Bergebeutel 2 frei. Wenn das Hüllrohr 9 ganz vom Bügel 5 abgeschoben ist (vgl. Fig. 2), so hat sich der Bügel 5 entfaltet und der Bergebeutel 2 zusammen mit dem in ihm angeordneten vom Netz 7 gebildeten Beutel kann sich ausrollen (Fig. 2).

Zum Verschieben des Hüllrohrs 9 kann an diesem ein nach außen abstehender Vorsprung 10 angeordnet sein, der beim Einführen des Instruments in den Trokar an den Rand des Trokars zur Anlage kommt, wodurch das Hüllrohr 9 beim weiteren Einschieben nach proximal verschoben wird.

In dem in den Figuren 2 bis 4 dargestellten Einfüllzustand, in welchem die Einfüllöffnung des Bergebeutels 2 geöffnet ist und mit diesem der vom Netz 7 gebildete Beutel geöffnet ist und eine gemeinsame insgesamte Einfüllöffnung ausbildet, kann aus dem Körper bei einer endoskopischen Operation zu entfernendes Material 11 in den Bergebeutel 2 und in den in diesem angeordneten vom Netz 7 gebildeten Beutel eingebracht werden. Ein solches Material 11 ist in Fig. 3 schematisch eingezeichnet. Das Material 11 ist hierbei nicht am in Fig. 3 unten liegenden Ende des Bergebeutels 2 dargestellt, da der Bergebeutel im Körper beispielsweise auch horizontal angeordnet sein kann, d.h. die Einfüllöffnung 4 weist nicht nach oben sondern seitlich.

Beim Material 11 kann es sich insbesondere um organisches Material handeln. Solches im Rahmen einer endoskopischen Operation aus dem Körper zu entfernendes Material wird auch als Resektat oder Spezimen bezeichnet.

Die Einfüllöffnung 4 des Bergebeutels 2 kann zusammengezogen werden. Hierzu verläuft ein längserstrecktes Zugelement 12, beispielsweise ein Faden oder ein Seil oder ein Band durch einen den Bergebeutel 2 in der Nähe seiner Einfüllöffnung 4 umgebenden Kanal 13. Im Anschluss an ihren Verlauf durch den Kanal 13 sind die beiden Enden des Zugelements 12 in einen Kanal 14 im Schaft 1 hineingeführt und erstrecken sich durch diesen und können mittels eines Betätigungsteils 15 gegenüber dem Schaft 1 verschoben werden. Hierbei wird die vom Zugelement 12 gebildete, durch den Kanal 13 verlaufende Schlaufe zusammengezogen, wobei der Bergebeutel 2 ausgehend von einem dem Schaft 1 gegenüberliegenden distalen Abschnitt des Bügels 5 vom Bügel 5 zunehmend abgerissen wird. Bei diesem Abreißen wird der Kanal 6 aufgerissen. Zur Ermöglichung bzw. Erleichterung dieses Aufreißens ist vorzugsweise eine geschwächte linienförmige Zone bzw. Reißnaht 16 im Material des Bergebeutels 2, welches den Kanal 6 begrenzt, ausgebildet. Diese Reißnaht 16 verläuft vorzugsweise im Bereich der Außenseite des Bügels 5.

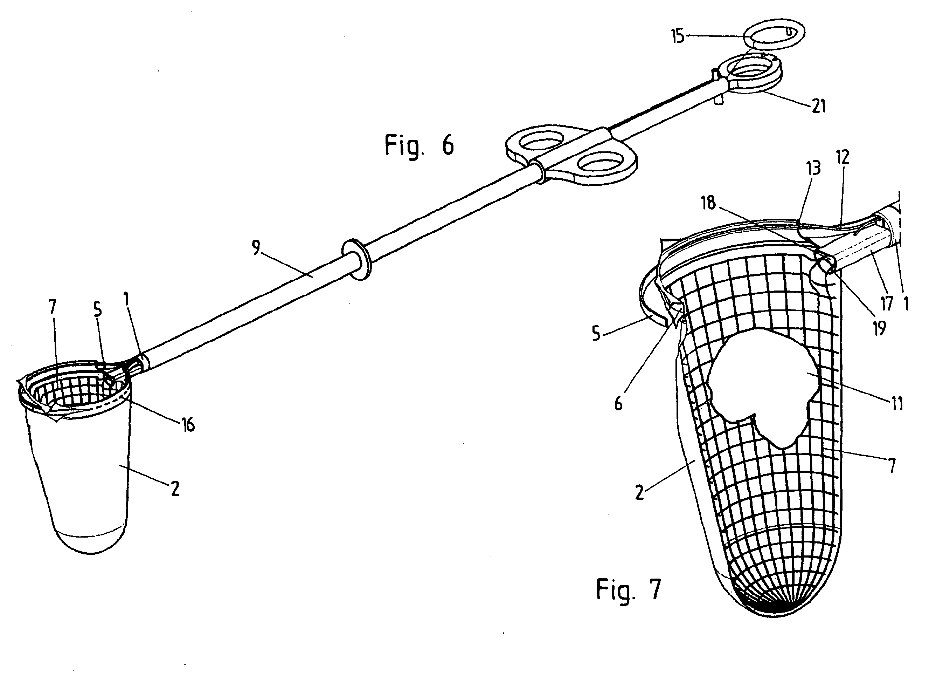

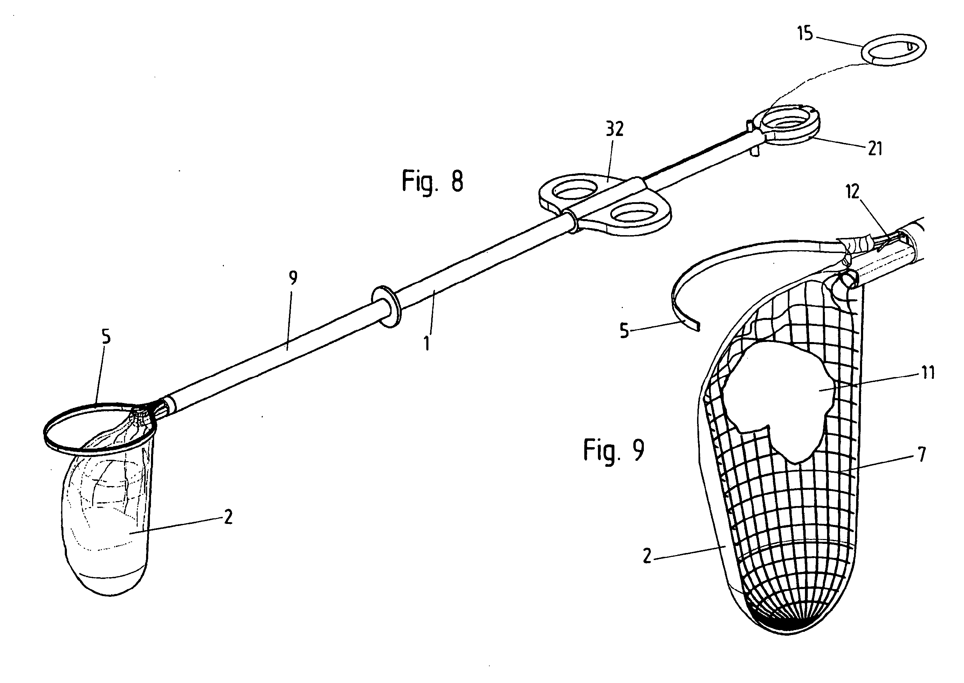

In den Figuren 6 und 7 ist ein Zustand zu Beginn des Abreißens des Bergebeutels 2 vom Bügel 5 dargestellt. In den Figuren 8 und 9 ist der Endzustand des Schließens des Bergebeutels 2 dargestellt. Hierbei ist die vom Zugelement 12 gebildete Schlaufe weitgehend zusammengezogen (ihr in Richtung der Längserstreckung des Schaftes 1 gemessener Durchmesser hat sich auf weniger als ein Drittel, vorzugsweise weniger als ein fünftel, des ursprünglichen Wertes verringert) und der Bergebeutel 2 ist weitgehend vom Bügel 5 abgerissen (über mehr als 80 % der ursprünglichen Länge des Kanals 6). Um ein vollständiges Abreißen des Bergebeutels 2 vom Bügel 5 zu verhindern kann beispielsweise die Reißnaht 16 an der gewünschten Endstelle des Abreißens enden.

Im nunmehr geschlossenen Zustand des Bergebeutels 2 ist die Einfüllöffnung 4 des Bergebeutels 2 zusammengezogen und über einen Endabschnitt 17 des Schaftes 1 gezogen. Der Endabschnitt 17 des Schaftes 1 ragt somit durch die zusammengezogene Einfüllöffnung des Bergebeutels 2 in diesen hinein und die verbleibende Einfüllöffnung 4 (Restöffnung) wird durch den in sie hineinragenden Endabschnitt 17 des Schafts 1 ausgefüllt und somit geschlossen.

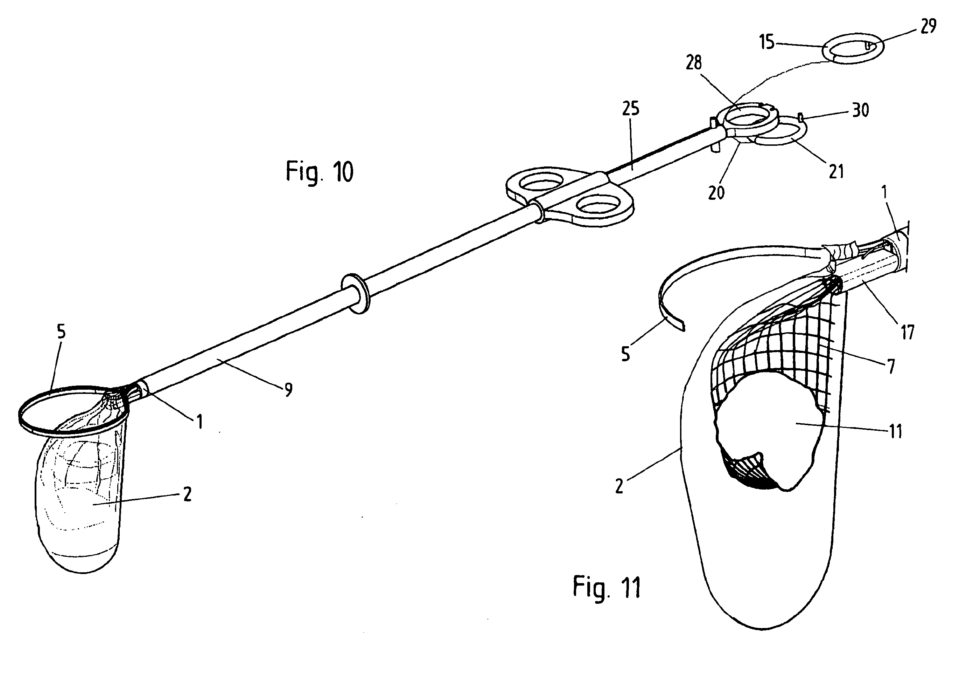

Am stirnseitigen distalen Ende des Endabschnitts 17 des Schafts 1 mündet ein Kanal, welcher einen inneren Hohlraum 18 des Schafts 1 bildet, wie dies am Besten aus Figur 5 ersichtlich ist. Vorzugsweise weist der Hohlraum 18 im Mündungsbereich einen Durchmesser bzw. eine lichte Weite von weniger als 0,5 cm auf. In diesen Hohlraum 18 verläuft mindestens ein am Netz 7 angreifendes längserstrecktes Zugelement 19. Im gezeigten Ausführungsbeispiel sind mehrere solche Zugelemente 19 vorhanden (vgl. insbesondere Fig. 4), die von verlängerten Filamenten des Netzes 7 gebildet werden. Die Zugelemente 19 sind innerhalb des Hohlraums 18 an einem gemeinsamen Zugelement 20 angebracht, welches mittels eines Betätigungsteils 21 gegenüber dem Schaft 1 verschoben werden kann. Es könnten auch die Zugelemente 19 bis zum Betätigungsteil 21 verlaufen. Dadurch kann das Netz 7 mittels des Betätigungsteils 21 nach und nach in den Hohlraum 18 des Schaftes 1 hineingezogen werden. Hierbei wird zunächst der vom Netz gebildete Beutel geschlossen, indem der die Öffnung aufweisende Teil dieses Beutels in den Hohlraum 18 hineingezogen wird. Der aus dem Hohlraum 18 herausragende Teil des vom Netz 7 gebildeten Beutels wird somit zunehmend verkleinert. In den Figuren 10 und 11 ist eine Zwischenstellung dargestellt, in welcher das Netz teilweise in den Hohlraum 18 hineingezogen ist.

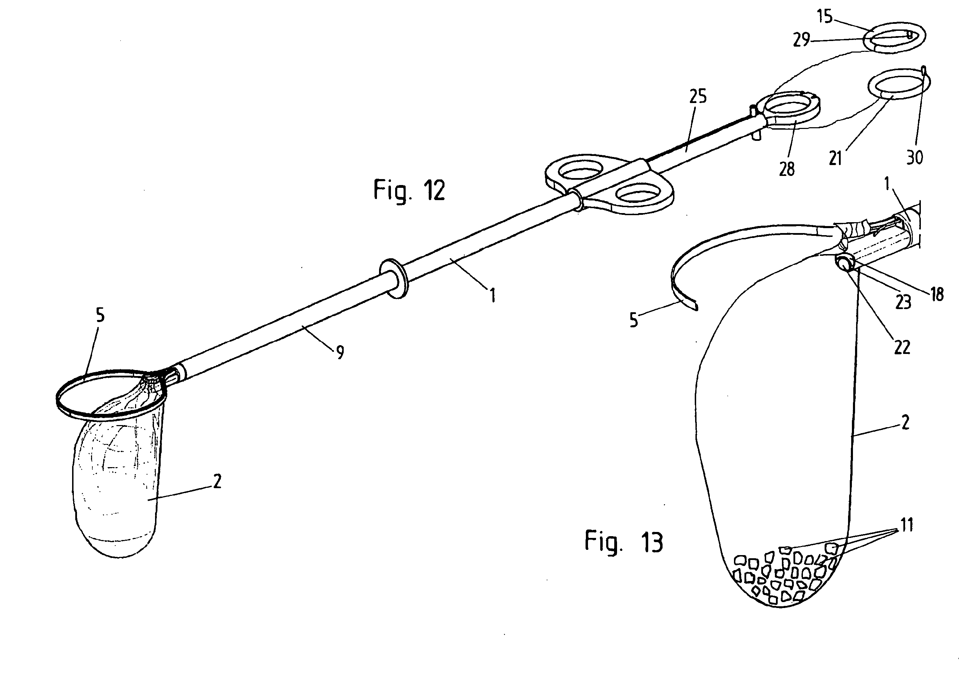

Wenn das Netz 7 soweit in den Hohlraum 18 hineingezogen worden ist, dass im vom Netz 7 gebildeten Beutel sich befindendes Material 11 nicht weiter in Richtung zum distalen Ende des Endabschnitts 17 gezogen werden kann und aufgrund seiner Abmessungen auch nicht in den Hohlraum 18 hineingezogen werden kann, so beginnen die Filamente des Netzes 7 beim weiteren Hineinziehen des Netzes 7 in den Hohlraum 18 das Material 11 zu durchschneiden, wobei die aufgeschnittenen Teile des Materials 11 durch die Maschen des Netzes gedrückt werden. Wenn das Netz 7 vollständig in den Hohlraum 18 hineingezogen worden ist, vgl. Fig. 12 und 13, so ist das Netz durch das Material 11 hindurch gezogen worden und das Material 11 ist entsprechend zerkleinert.

In der Endstellung ist das Netz 7, welches sich in der Anfangsstellung im Bergebeutel 2 befunden hat, vollständig aus dem Bergebeutel 2 herausgezogen. Anstatt das Netz in einen Hohlraum 18 im Schaft 1 oder in einem mit diesem verbundenen Teil hineinzuziehen, wäre es beispielsweise auch denkbar und möglich, das Netz durch die Öffnung eines mit dem Schaft 1 verbundenen Rings durchzuziehen, wobei es aus dem Bergebeutel 2 herausgezogen wird.

Im gezeigten Ausführungsbeispiel schließt der Endabschnitt 17 über eine Stufe an den weiter proximal gelegenen Teil des Schaftes an, welcher gegenüber dem Endabschnitt 17 einen größeren Durchmesser aufweist. In der Stufe mündet der Kanal 14 für das Zugelement 12. Auch andere Ausbildungen sind denkbar und möglich.

Die zerkleinerten Teile des Materials 11 können in der Folge mittels einer Saugleitung 22 abgesaugt werden. Die Saugleitung 22 befindet sich zunächst in einem weiteren Kanal 23 im Schaft 1, welcher am distalen stirnseitigen Ende des distalen Endabschnitts 17 des Schafts 1 mündet. Der Kanal 23 und der den Hohlraum 18 bildende Kanal können durch eine Wand 24 (vgl. Fig. 5) voneinander abgegrenzt sein.

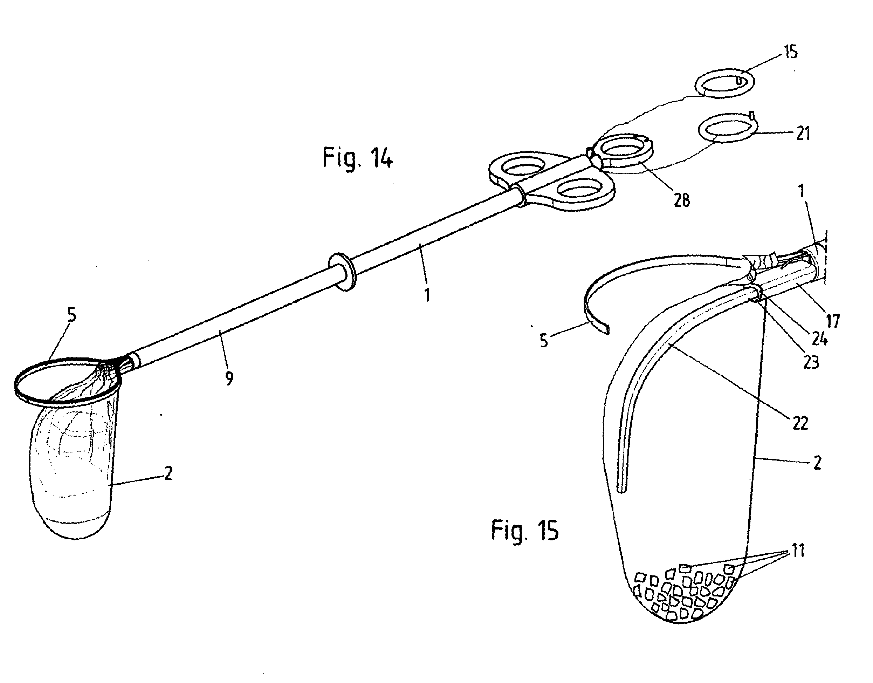

Die Saugleitung 22 kann aus dem Kanal 23 herausgeschoben werden, wie dies in den Figuren 14 und 15 dargestellt ist. Beispielsweise kann hierzu ein Rohrstück 25, an dem die Saugleitung 22 angeschlossen ist oder durch welches die Saugleitung 22 führt, in ein proximales Ende des Schafts 1 eingeschoben werden. Zumindest der aus dem distalen Ende des Schaftes 1 herausschiebbare Abschnitt der Saugleitung 22 besteht aus einem flexiblen Material.

Um ein Nachspülen von Flüssigkeit oder auch Gas in den geschlossenen Bergebeutel 2 zu ermöglichen, kann vorzugsweise mindestens ein Spülkanal zusätzlich zum mindestens einen Absaugkanal in die Saugleitung 22 integriert sein. Anschlussstutzen 26, 27 für den mindestens einen Saugkanal und den mindestens einen Spülkanal sind schematisch dargestellt.

Der Bergebeutel 2 kann somit durch wechselweises Saugen und Spülen vollständig oder zumindest teilweise geleert werden. Der derart vollständig oder zumindest teilweise geleerte Bergebeutel 2 kann problemlos durch den Trokar durchgezogen werden oder zusammen mit dem Trokar aus dem Körper entfernt werden.

Die Filamente des Netzes 7 werden aus einem dünnen (sodass ein leichtes Durchschneiden des Materials 11 ermöglicht wird) und ausreichend reißfesten flexiblen Material gebildet. Beispielsweise besteht das Netz aus Metall- oder Nylonfäden oder aus Kohlefasern.

Die Maschenweite des Netzes 7 beträgt vorzugsweise weniger als 1 cm.

Der Bergebeutel 2 besteht aus einem geeigneten flexiblen Material, wie dies von herkömmlichen Bergebeuteln her bekannt ist, z.B. aus Polyurethan.

Denkbar und möglich ist es auch, dass das Netz 7 nur einen Teil des innerhalb des Bergebeutels 2 angeordneten Beutels ausbildet, beispielsweise nur den Boden im Bereich des geschlossenen Endes 8 dieses Beutels bildet.

Das Netz 7 könnte auch in mehrere Hohlräume 18 im Schaft oder in einem mit diesem verbundenen Teil hineingezogen werden (unter Auftrennung des Netzes) oder durch mehrere mit dem Schaft verbundenen Ringe durchgezogen werden.

Es könnte auch eine separate Einrichtung zum Schließen des vom Netz 7 gebildeten oder dieses aufweisenden Beutels vorhanden sein, beispielsweise ein Zugelement, welches durch einen Kanal dieses Beutels verläuft, welcher den Beutel im Bereich seiner Einfüllöffnung umgibt.

Die in das medizinische Instrument integrierte Saugleitung 22 (bzw. Saug-Spülleitung 22) könnte auch entfallen und das zerkleinerte Material 11 könnte mittels eines separaten Sauginstruments aus dem Bergebeutel 2 abgesaugt werden.

In einer weiteren möglichen Ausführungsform der Erfindung könnte der Bergebeutel 2 auch im Bereich seines geschlossenen Endes 3 mit einem Fortsatz mit einem verringerten Durchmesser versehen werden. In diesen "wurmartigen" Fortsatz könnte das mittels des Netzes 7 zerkleinerte Material fallen. Dieser wurmartige Fortsatz könnte hierbei einen so kleinen Durchmesser aufweisen, dass das medizinische Instrument mit dem in diesen Fortsatz gefallenen zerkleinerten Material durch den Trokar herausgezogen werden kann oder zusammen mit dem Trokar aus dem Körper herausgezogen werden kann.

Die Betätigungsteile 15, 21 für den Bergebeutel 2 und das Netz 7 sind im gezeigten Ausführungsbeispiel in Form von Ringen dargestellt. In ihrer Anfangsstellung sind sie an einem proximal am Rohrstück 25 angeordneten Ring 28 gehalten, beispielsweise mittels Stiften 29, 30 in diesen eingerastet. Diese Betätigungsteile 15, 21 können auch in unterschiedlicher anderer Art und Weise ausgebildet sein, beispielsweise in Form von Betätigungshebeln oder Drehgriffen. Auch unterschiedliche Ausbildungen für das Betätigungsteil 15 und für das Betätigungsteil 21 sind möglich, beispielsweise um eine größere Kraft für das Zurückziehen des Netzes 7 aufbringen zu können. Die Betätigungsteile 15, 21 können am Schaft 1 oder an einem mit diesem starr oder - wie im gezeigten Ausführungsbeispiel - verschiebbar verbundenen Teil angeordnet sein.

Denkbar und möglich wäre es auch, dass das mindestens eine Zugelement 19 zum Herausziehen des vom Netz 7 gebildeten oder dieses aufweisenden Beutels aus dem Bergebeutel 2 nicht durch die Einfüllöffnung 4 des Bergebeutels 2 verläuft. Der Bergebeutel 2 könnte eine separate seitliche Öffnung aufweisen, durch welche dieses mindestens eine Zugelement 19 verläuft und der vom Netz 7 gebildete oder dieses aufweisende Beutel herausgezogen wird.

Weiters ist es denkbar und möglich, den Bergebeutel 2 nicht durch ein Zugelement 12 zu verschließen sondern indem das Hüllrohr 9 wieder nach distal verschoben wird, wobei der Bergebeutel 2 auf dem Bügel 5 zusammengeschoben wird und sich die Einfüllöffnung 4 schließt, wie dies bei Bergebeuteln nach dem Stand der Technik bekannt ist.

Das medizinische Instrument gemäß der Erfindung kann zum Bergen von Material aus dem menschlichen oder tierischen Körper (= Entfernen des Materials aus dem Körper, so dass es möglichst nicht mit anderen Körperteilen in Berührung kommt) bei verschiedenen Arten von endoskopischen Operationen eingesetzt werden, z.B. laparoskopischen Operationen oder intraluminalen endoskopischen Operationen, die auch unter dem Begriff NO-TES bekannt sind. Zum Einsatz bei solchen flexiblen endoskopischen Operationen können der Schaft sowie die an diesem distal angeordneten Teile flexibel ausgebildet sein. Das Netz 7 eignet sich bei den verschiedenen Arten der endoskopischen Operationen zum Zerkleinern von ausreichend weichem Material, insbesondere Körpermaterial.

Bei einem erfindungsgemäßen Instrument mit einem innerhalb des Bergebeutels angeordneten Netz 7 zum Zerteilen von in den Bergebeutel 2 eingebrachtem Material 11 könnte der Bergebeutel 2 auch in anderer Weise als im gezeigten Ausführungsbeispiel beschrieben ausgebildet sein. So könnte der Bergebeutel 2, wie dies aus dem Stand der Technik bekannt ist, beispielsweise auch eine in eine distale Richtung weisende verschließbare Einfüllöffnung aufweisen. Der vom Netz 7 gebildete oder das Netz 7 aufweisende innerhalb des Bergebeutels 2 angeordnete Beutel könnte in diesem Fall ebenfalls eine in distale Richtung weisende verschließbare Öffnung aufweisen und von seinem proximalen Ende her in einen Hohlraum des Schaftes hineinziehbar sein.

### Legende

### zu den Hinweisziffern:

- 1: Schaft
- 2: Bergebeutel
- 3: geschlossenes Ende
- 4: Einfüllöffnung
- 5: Bügel
- 6: Kanal
- 7: Netz
- 8: geschlossenes Ende
- 9: Hüllrohr
- 10: Vorsprung
- 11: Material
- 12: Zugelement
- 13: Kanal
- 14: Kanal
- 15: Betätigungsteil
- 16: Reißnaht
- 17: Endabschnitt
- 18: Hohlraum
- 19: Zugelement
- 20: Zugelement
- 21: Betätigungsteil
- 22: Saugleitung
- 23: Kanal
- 24: Wand
- 25: Rohrstück
- 26: Anschlussstutzen
- 27: Anschlussstutzen
- 28: Ring
- 29: Stift
- 30: Stift
- 31: proximales Ende
- 32: Griff

## Patentansprüche

1. Medizinisches Instruments, welches einen Bergebeutel (2) zum Bergen von bei einer Operation, insbesondere endoskopischen Operation, aus einem zu operierenden Körper zu entfernenden Material (11) und in einer Anfangsstellung innerhalb des Bergebeutels (2) angeordnetes Mittel (7) zum Zerkleinern des Materials aufweist, wobei das medizinische Instrument mindestens ein Zugelement (19), das am Mittel (7) angreift, und ein mit dem Zugelement (19) zusammenwirkendes Betätigungsteil (21) aufweist, **dadurch gekennzeichnet, dass** das Mittel (7) zum Zerkleinern des Materials ein von einem Netz (7) gebildeter oder ein Netz (7) aufweisender Beutel ist, wobei das medizinische Instrument zum Durchziehen des Netzes (7) durch das zerkleinernde Material (11) mittels mindestens eines Zugelements (19) so ausgestattet ist, dass der vom Netz gebildete oder das Netz aufweisende Beutel, der sich in einer Anfangsstellung innerhalb des Bergbeutels (2) befindet, nach dem Durchziehen des Netzes (7) durch das zu zerkleinernde Material (11) zumindest teilweise aus dem Bergebeutel (2) herausgezogen ist.

2. Medizinisches Instrument nach Anspruch 1, **dadurch gekennzeichnet, dass** das medizinische Instrument mindestens einen inneren Hohlraum (18) aufweist, in den der vom Netz (7) gebildete oder das Netz (7) aufweisende Beutel zum Zerkleinern des zunächst in diesem Beutel sich befindenden Materials (11) zumindest teilsweise, vorzugsweise vollständig, hineinziehbar ist.

3. Medizinisches Instrument nach Anspruch 2, **dadurch gekennzeichnet, dass** der mindestens eine innere Hohlraum (18) in einem Schaft (1) des medizinischen Instruments angeordnet ist, durch den das mindestens eine Zugelement (19) verläuft.

4. Medizinisches Instrument nach Anspruch 3, **dadurch gekennzeichnet, dass** auf dem Schaft (1) ein Hüllrohr (9) angeordnet ist, welches von einer Ausgangsstellung, in welcher sich der Bergebeutel (2) innerhalb des Hüllrohrs (9) befindet, in Richtung zum proximalen Ende des medizinischen Instruments bis in eine Endstellung verschiebbar ist, in welcher sich der Bergebeutel (2) außerhalb des Hüllrohrs (9) befindet.

5. Medizinisches Instrument nach einem der Ansprüche 1 bis 4, **dadurch gekennzeichnet, dass** der Bergebeutel (2) einen Einfüllzustand zum Einfüllen des zu bergenden Materials (11) aufweist, in welchem eine Einfüllöffnung (4) des Bergebeutels (2) geöffnet ist, wobei im Einfüllzustand des Bergebeutels (2) auch der vom Netz (7) gebildete oder das Netz (7) aufweisende Beutel zur gleichen Seite hin wie der Bergebeutel (2) geöffnet ist, und dass zum Schließen des Bergebeutels (2) die Einfüllöffnung (4) des Bergebeutels (2) schließbar ist.

6. Medizinisches Instrument nach Anspruch 5, **dadurch gekennzeichnet, dass** zum Schließen des Bergebeutels (2) durch Zusammenziehen der Einfüllöffnung (4) des Bergebeutels (2) ein in einem Kanal (13) um den Bergebeutel (2) im Bereich von dessen Einfüllöffnung (4) verlaufendes Zugelement (12) mittels eines Betätigungsteils (15) zusammenziehbar ist.

7. Medizinisches Instrument nach Anspruch 6, **dadurch gekennzeichnet, dass** der Bergebeutel (2) in seinem Einfüllzustand an einem Bügel (5) aus einem federelastischen Material aufgehängt ist und der Bergebeutel (2) zum Schließen des Bergebeutels (2) mittels des mindestens einen Zugelements (12) vom Bügel (5) ausgehend von einem distalen Abschnitt des Bügels (5) zunehmend abreißbar ist.

8. Medizinisches Instrument nach einem der Ansprüche 5 bis 7, **dadurch gekennzeichnet, dass** im geschlossenen Zustand des Bergebeutels (2) in eine Restöffnung der zusammengezogenen Einfüllöffnung (4) des Bergebeutels (2) ein distaler Endabschnitt (17) des Schaftes (1) ragt, wobei am distalen Endabschnitt (17) des Schaftes (1), vorzugsweise stirnseitig, mindestens ein Hohlraum (18) zum Hineinziehen des Netzes (7) mündet.

9. Medizinisches Instrument nach einem der Ansprüche 1 bis 10, **dadurch gekennzeichnet, dass** das medizinische Instrument eine Saugleitung (22) aufweist, welche zum Absaugen von mittels des Netzes (7) zerkleinertem Material (11) in den Bergebeutel (2) einführbar ist.

10. Medizinisches Instrument nach Anspruch 9, **dadurch gekennzeichnet, dass** die Saugleitung (22) durch einen Kanal (23) im Schaft (1) verläuft und gegenüber dem Schaft (1) verschiebbar ist.

11. Medizinisches Instrument nach Anspruch 10, **dadurch gekennzeichnet, dass** der die Saugleitung (22) aufnehmende Kanal (23) am distalen Endabschnitt (17) des Schaftes (1) stirnseitig mündet.

## Claims

1. A medical instrument which has a retrieval bag (2) for retrieving material (11) to be removed during an operation, in particular an endoscopic operation, from a body to be operated on and a means (7), arranged in an initial position thereof within the retrieval bag (2), for breaking up the material, wherein the medical instrument has at least one pulling element (19) which acts upon the means (7) and an actuating part (21) which cooperates with the pulling element (19), **characterised in that** the means (7) for breaking up the material is a bag formed by a net (7) or having a net (7), wherein for drawing the net (7), by means of at least one pulling element (19), through the material (11) to be broken up, the medical instrument is equipped in such a manner that the bag, formed by the net or having the net, located in an initial position thereof within the retrieval bag (2) is located at least partially drawn out of the retrieval bag (2) after the net (7) has been drawn through the material (11) to be broken up.

2. A medical instrument according to claim 1, **characterised in that** the medical instrument has at least one inner cavity (18) into which the bag, formed by the net (7) or having the net (7), for breaking up the material (11) initially located in this bag can be drawn at least partially, preferably completely.

3. A medical instrument according to claim 2, **characterised in that** the at least one inner cavity (18) is arranged in a shaft (1), through which there runs the at least one pulling element (19), of the medical instrument.

4. A medical instrument according to claim 3, **characterised in that** a jacket tube (9) is arranged on the shaft (1) and can be displaced in the direction of the proximal end of the medical instrument from an initial position in which the retrieval bag (2) is located within the jacket tube (9) into an end position in which the retrieval bag (2) is located exterior to the jacket tube (9).

5. A medical instrument according to any one of claims 1 to 4, **characterised in that** the retrieval bag (2) has a filling state for filling the material (11) to be retrieved into the retrieval bag (2), in which a filling opening (4) of the retrieval bag (2) is open, wherein in the filling state of the retrieval bag (2), the bag formed by the net (7) or having the net (7) is also open to the same side as the retrieval bag (2), and **in that** the filling opening (4) of the retrieval bag (2) is closable to close the retrieval bag (2).

6. A medical instrument according to claim 5, **characterised in that** to close the retrieval bag (2) by drawing together of the filling opening (4) of the retrieval bag (2), a pulling element (12) running in a channel (13) around the retrieval bag (2) in the region of the filling opening (4) thereof can be drawn together by means of an actuating part (15).

7. A medical instrument according to claim 6, **characterised in that** the retrieval bag (2) is in its filling state suspended on a loop (5) of a resilient material and, in order to close the retrieval bag (2), the retrieval bag (2) can be progressively torn off from the loop (5), starting from a distal portion of the latter, by means of the at least one pulling element (12).

8. A medical instrument according to any one of claims 5 to 7, **characterised in that** in the closed state of the retrieval bag (2), a distal end portion (17) of the shaft (1) projects into a remaining opening of the drawn-together filling opening (4) of the retrieval bag (2), wherein at least one cavity (18) for drawing in the net (7) issues at the distal end portion (17) of the shaft (1), preferably at the end face thereof.

9. A medical instrument according to any one of claims 1 to 10, **characterised in that** the medical instrument has a suction line (22) insertable into the retrieval bag (2) in order to draw off material (11) broken down by means of the net (7).

10. A medical instrument according to claim 9, **characterised in that** the suction line (22) runs through a channel (23) in the shaft (1) and is displaceable with respect to the shaft (1).

11. A medical instrument according to claim 10, **characterised in that** the channel (23) receiving the suction line (22) issues at the end face of the distal end portion (17) of the shaft (1).

## Revendications

1. Instrument médical, comportant une poche de collecte (2) pour collecter la matière (11) à retirer du corps à opérer pendant une opération, en particulier une opération endoscopique, et un moyen (7) pour fractionner la matière, disposé à l'intérieur de la poche de collecte (2) dans une position initiale, l'instrument médical comportant au moins un élément de traction (19), intervenant sur le moyen (7), et un élément de manoeuvre (21) coopérant avec l'élément de traction (19), **caractérisé en ce que** le moyen (7) pour fractionner la matière est une poche formée par une grille (7) ou munie d'une grille (7), l'instrument médical, afin de tirer la grille (7) au moyen d'au moins un élément de traction (19) à travers la matière (11) à fractionner, étant configuré de telle sorte que la poche, formée par la grille ou munie de la grille et située à l'intérieur de la poche de collecte (2) dans une position initiale, est tirée au moins en partie hors de la poche de collecte (2) après que la grille (7) a été tirée à travers la matière (11) à fractionner.

2. Instrument médical selon la revendication 1, **caractérisé en ce que** l'instrument médical comporte au moins une cavité (18) intérieure, dans laquelle peut être tirée au moins en partie, de préférence en totalité, la poche formée par la grille (7) ou munie de la grille (7) et destinée à fractionner la matière (11) située d'abord dans ladite poche.

3. Instrument médical selon la revendication 2, **caractérisé en ce que** ladite au moins une cavité (18) intérieure est disposée dans une tige (1) de l'instrument médical, à travers laquelle s'étend ledit au moins un élément de traction (19).

4. Instrument médical selon la revendication 3, **caractérisé en ce que** sur la tige (1) est disposée une gaine tubulaire (9) pouvant coulisser depuis une position initiale, dans laquelle la poche de collecte (2) se situe à l'intérieur de la gaine tubulaire (9), vers l'extrémité proximale de l'instrument médical jusque dans une position finale, dans laquelle la poche de collecte (2) se situe à l'extérieur de la gaine tubulaire (9).

5. Instrument médical selon l'une quelconque des revendications 1 à 4, **caractérisé en ce que** la poche de collecte (2) comporte une position de remplissage pour introduire la matière (11) à collecter, dans laquelle une ouverture de remplissage (4) de la poche de collecte (2) est ouverte, la poche formée par la grille (7) ou munie de la grille (7) étant également ouverte dans la position de remplissage de la poche de collecte (2) vers le même côté que la poche de collecte (2), et **en ce qu'**on peut fermer l'ouverture de remplissage (4) de la poche de collecte (2) pour fermer la poche de collecte (2).

6. Instrument médical selon la revendication 5, **caractérisé en ce que** pour fermer la poche de collecte (2) par le resserrement de l'ouverture de remplissage (4) de la poche de collecte (2), il est possible de resserrer au moyen d'un élément d'actionnement (15) un élément de tirage (12) s'étendant dans un canal (13) autour de la poche de collecte (2) dans la zone de l'ouverture de remplissage (4) de cette dernière.

7. Instrument médical selon la revendication 6, **caractérisé en ce que** dans sa position de remplissage, la poche de collecte (2) est suspendue à un arceau (5) en matériau flexible, et, pour fermer la poche de collecte (2), ladite poche de collecte (2) peut être détachée de plus en plus de l'arceau (5) à partir d'une partie distale de l'arceau (5) au moyen dudit au moins un élément de tirage (12).

8. Instrument médical selon l'une quelconque des revendications 5 à 7, **caractérisé en ce que** dans la position fermée de la poche de collecte (2), une extrémité distale (17) de la tige (1) s'engage dans une ouverture restante de l'ouverture de remplissage (4) resserrée de la poche de collecte (2), au moins une cavité (18) débouchant à l'extrémité distale (17) de la tige (1), de préférence du côté frontal, pour y tirer la grille (7).

9. Instrument médical selon l'une quelconque des revendications 1 à 10, **caractérisé en ce que** l'instrument médical comporte une conduite d'aspiration (22), qui peut être insérée dans la poche de collecte (2) pour aspirer la matière (11) fractionnée au moyen de la grille (7).

10. Instrument médical selon la revendication 9, **caractérisé en ce que** la conduite d'aspiration (22) s'étend dans la tige (1) à travers un canal (23) et peut coulisser par rapport à la tige (1).

11. Instrument médical selon la revendication 10, **caractérisé en ce que** le canal (23) recevant la conduite d'aspiration (22) débouche du côté frontal au niveau de l'extrémité distale (17) de la tige (1).
